# EUROPEAN PATENT APPLICATION

(11) **EP 3 926 343 A1**
(43) Date of publication of application: **22.12.2021**
(21) Application number: 20754901.5
(22) Date of filing: 12.02.2020
(51) Int. Cl.: G01N 33/86

(54) **HEMOSTATIC ENZYME AND CARBOXYMETHYL CHITOSAN-CONTAINING COMPOSITION FOR BLOOD COAGULATION TEST, AND USE THEREOF**

(30) Priority: 14.02.2019 KR 20190017080; 11.02.2020 KR 20200016467
(71) Applicant: NC Bit Inc., Seongnam-si, Gyeonggi-do 13488 (KR)
(72) Inventor: KO, Eunhye, Seoul 02840 (KR); WOO, Yongje, Bucheon-si Gyeonggi-do 14597 (KR); KIM, Jong-Tak, Seoul 06082 (KR); SEOMUN, Young, Seoul 08734 (KR); CHO, Kil Sang, Seongnam-si Gyeonggi-do 13583 (KR); KIM, Kyoung Soo, Seongnam-si Gyeonggi-do 13460 (KR)
(74) Representative: Mewburn Ellis LLP
(86) International application number: PCT/KR2020/001961
(87) International publication number: WO 2020/166961

(57) **Abstract**

The present invention relates to a composition for blood coagulation testing and the use thereof, and more particularly to a composition for blood coagulation testing containing a hemostatic enzyme and carboxymethyl chitosan and the use thereof. The composition for blood coagulation testing according to the present invention is useful because it may perform a blood coagulation test with high sensitivity and high speed by improving the activity of the hemostatic enzyme and clot strength and increasing the rate of fibrin formation.

## Description

### Technical Field

The present invention relates to a composition for blood coagulation testing and the use thereof, and more particularly to a composition for blood coagulation testing containing a hemostatic enzyme and carboxymethyl chitosan and the use thereof.

### Background Art

Fibrinogen and thrombin are critical proteins involved in hemostasis after vascular injury and are essential for blood clot formation. Fibrinogen and thrombin can be combined in powder form or in a nonaqueous suspension, without initiating a typical clotting reaction, thus preventing the formation of a fibrin clot until these proteins are hydrated in an aqueous medium or other liquid environment in which the proteins are soluble. A mixture of these proteins in powder form has a variety of potential biomedical applications including topical hemostasis, tissue repair, drug delivery, etc. In addition, a mixture of these proteins may be loaded onto a carrier or substrate, or other medical device, in powder form to form a product that may be used, for example, as a hemostatic device.

Fibrinogen is most frequently measured by a method originally described by Clauss, which measures fibrinogen functionality based on the rate of clot formation. In a typical Clauss assay, a sample containing an unknown amount of soluble fibrinogen is blended with an excess of thrombin. In a typical Clauss assay, a sample containing an unknown amount of soluble fibrinogen is combined with an excess of thrombin. The proportions of fibrinogen and thrombin are such that fibrinogen is the rate limiting reactant and the rate of clot formation is a function of the fibrinogen concentration. A fast clotting time would be indicative of a high fibrinogen concentration. Conversely, a longer clotting time would indicate a low concentration of functional fibrinogen. The amount of functional fibrinogen can be quantified by comparing the clotting time of the sample with those of a series of standards to establish a standard curve. The concentration of fibrinogen in the sample can be determined mathematically based on the equation derived from the clotting times of the standards.

Fibrinogen-C reagent (Instrument Lab.) is a diagnostic reagent capable of detecting the concentration of fibrinogen according to the above-described principle, but has a problem of low sensitivity.

Meanwhile, the effects of snake venom on blood clotting and thrombolytic systems in mammals including humans have been studied for a long period of time, and many active ingredients have been isolated from snake venom and the activities thereof have been investigated. Various components constituting snake venom are known to function as pro-coagulants or anticoagulants by directly or indirectly affecting fibrin clotting or platelet aggregation (Meaume, J. Toxicon, 4:2558 (1966); Matsuiet al., Biochim. Biophys. Acta., 1477: 146-156 (2000)). Some of these components have been widely used in the diagnosis and treatment of thrombosis. Thereamong, more than 20 types of thrombin-like enzymes, which convert fibrinogen into fibrin by cleaving fibrinopeptides, have been reported to date, and cDNA sequences of some thereof have been identified.

The thrombin-like enzyme initially hydrolyzes fibrinopeptide A of a fibrinogen molecule to form an unstable fibrin clot (called des-A-fibrin), unlike thrombin that is a mammalian native blood coagulation protein, but this unstable fibrin clot is rapidly degraded by *in vivo* fibrinolysis over time, resulting in a decrease in blood fibrinogen levels (Pirkle, H., and Stocker, K. Thromb. Haemost. 65:444-450(1991); Marsh, N.A., Blood Coagul. Fibrinolysis, 5:339-410(1994)).

In actual clinical practice, this enzyme with two-sided characteristics is used as a hemostatic agent and also used as an agent for preventing and treating thrombosis. In addition, this enzyme has the advantage of not affecting platelet activation and other coagulation factors in blood, and thus exhibits effective hemostatic activity when a small amount (2 NIH units/60 kg) of the enzyme is injected intramuscularly and intravenously 1 to 2 hours before surgery. Meanwhile, as the dosage and administration time of the enzyme are adjusted, the concentration of fibrinogen in the blood can be lowered without side effects such as bleeding that may occur when a thrombolytic enzyme is used. The release of des-A-fibrin and fibrinogen degradation products (FDPs) formed in this process stimulates vascular endothelial cells to induce the production of plasminogen activators and inhibits thrombin activity. Thus, the enzyme is also used to prevent and treat thrombosis (Schumacher et al., Thromb. Res. 81:187-194 (1996); and Bell W. R. Jr., Drugs, 54:18-30 (1997)).

All of the thrombin-like enzymes that are actually used in clinical practice are natural proteins isolated and purified from snake venom, the most representative of which is batroxobin isolated from the venom of Bothrops atrox moojeni, a Latin American venomous snake. Batroxobin is sold exclusively by Pentapharm (Switzerland), and is also commercially available under brand names such as Reptilase (for hemostasis), Defibrase (for thrombolysis), and Reptilase-reagent (for diagnostic reagent). In addition, botropase (for hemostasis, Ravizza, Italy) purified from the venom of Bothrops jararaca, a Latin American venomous snake, and Ancrod (Knoll Pharmaceuticals, USA) isolated from the venoms of the Malayan pit viper and *Calloselasma rhodostoma,* are also commercially available.

In addition, the Vivostat System (Vivosolution, Denmark) that uses batroxobin for the preparation of an autologous fibrin sealant for preventing bleeding during surgery and suturing has recently attracted attention.

Korean Patent No. 10-1901150 discloses a recombinant batroxobin-containing composition comprising glycosylated recombinant batroxobin and a hemostatic composition comprising the same, but a method of testing blood coagulation using the recombinant batroxobin is not known.

Accordingly, the present inventors have made extensive efforts to solve the above-described problems and develop a composition for blood coagulation testing, which has high sensitivity, and as a result, have found that, when the concentration of fibrinogen is measured using a composition containing a hemostatic enzyme and carboxymethyl chitosan, the concentration of fibrinogen may be detected with high sensitivity and accuracy, thereby completing the present invention.

### Summary of the Invention

An object of the present invention is to provide a composition for blood coagulation testing.

Another object of the present invention is to provide a method of measuring fibrinogen concentration using the composition.

Still another object of the present invention is to provide a method of screening an anticoagulant substance using the composition.

To achieve the above objects, the present invention provides a composition for blood coagulation testing, the composition containing a hemostatic enzyme and carboxymethyl chitosan (CMCS).

The present invention also provides a method for measuring fibrinogen concentration, the method comprising steps of: a) adding the composition to a serum-containing sample and measuring the rate of clot formation; and b) determining the fibrinogen concentration from the rate of clot formation.

The present invention also provides a method for screening an anticoagulant substance, the method comprising steps of: a) adding a hemostatic enzyme and carboxymethyl chitosan to a serum-containing sample containing fibrinogen to obtain a mixture; b) adding a candidate substance to the mixture; c) measuring the time of clot formation; and d) selecting the candidate substance as the anticoagulant substance when the time of clot formation increases compared to that in a control to which the candidate substance has not been added.

The present invention also provides the use of a composition containing a hemostatic enzyme and carboxymethyl chitosan (CMCS) for blood coagulation testing.

### Brief Description of Drawings

FIG. 1 depicts graphs showing the results of evaluating the synergistic effect of a combination of a hemostatic enzyme and carboxymethyl chitosan. FIG. 1(A) is a graph comparing the plasma clotting time; FIG. 1(B) is a graph comparing the fibrinogen clotting time; FIG. 1(C) is a graph showing the clotting time as a function of rTLH concentration; and FIG. 1(D) is a graph showing the clotting time as a function of CMCS concentration.
FIG. 2 shows the results of measuring the plasma clotting times (A) and fibrinogen clotting times (B) for various types of hemostatic enzymes (recombinant hemostatic enzyme (rTLH), native batroxobin (nBat), thrombin, Botropase, Ancrod, and Suling), and compares the enzyme alone with a composition containing the enzyme and carboxymethyl chitosan.
FIG. 3 shows the results of analyzing the time taken for the fibrinogen alpha chain (a-chain) to be cleaved by the recombinant hemostatic enzyme in the presence or absence of carboxymethyl chitosan.
FIG. 4 shows the results of measuring the activity of a recombinant hemostatic enzyme (rTLH) combined with carboxymethyl chitosan in the presence of a thrombin substrate. FIG. 4(A) is a graph showing the degree of activity of the recombinant hemostatic enzyme, and FIG. 4(B) is a table that summarizes the quantitatively measured values.
FIG. 5 shows the results of measuring the clotting strength of the recombinant hemostatic enzyme depending on the concentration of carboxymethyl chitosan. FIG. 5(A) is a graph showing the degree of clot formation, FIG. 5(B) is a table showing quantitative values.
FIG. 6 shows the results of measuring fibrinogen concentration using a recombinant hemostatic enzyme and carboxymethyl chitosan according to the present invention.
FIG. 7 shows the results of measuring fibrinogen concentration using a composition containing a recombinant hemostatic enzyme and carboxymethyl chitosan according to the present invention, which further contains calcium chloride.
FIG. 8 shows the results of detecting low concentrations of fibrinogen in serum using a composition containing a recombinant hemostatic enzyme and carboxymethyl chitosan according to the present invention, which further contains calcium chloride.
FIG. 9 shows the results of detecting fibrinogen concentration in plasma containing various amounts of hemoglobin, bilirubin and heparin, which may have effects on result values when measuring fibrinogen concentration using a composition containing a recombinant hemostatic enzyme and carboxymethyl chitosan according to the present invention.
FIG. 10 shows the results of confirming whether the fibrinogen value measured using a composition containing a recombinant hemostatic enzyme and carboxymethyl chitosan according to the present invention is consistent with the result value of an international standard substance.

### Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, it was confirmed that, when a blood coagulation test was performed using a composition containing a hemostatic enzyme and carboxymethyl chitosan, the blood coagulation test could be performed with high sensitivity and high speed.

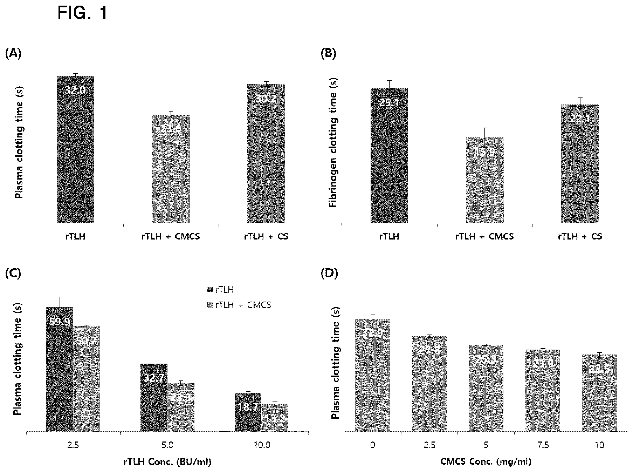

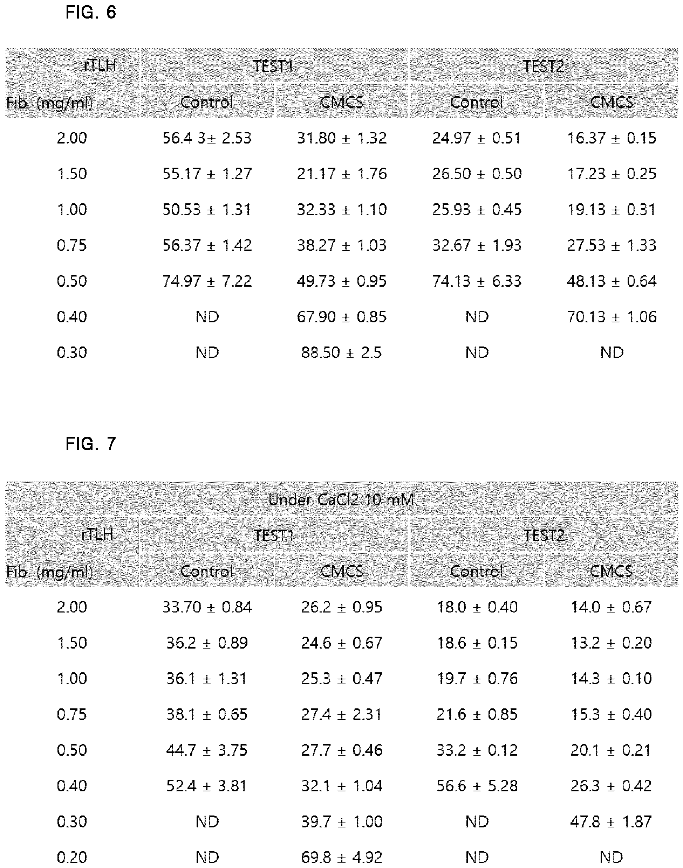

That is, in one example of the present invention, it was confirmed that carboxymethyl chitosan improved the enzymatic activity of a hemostatic enzyme and clot strength (FIGS. 1 to 5). Thus, it has been confirmed that, when fibrinogen concentration is measured using a composition containing a thrombin-like enzyme (TLE) and carboxymethyl chitosan, it is possible to detect a lower concentration of fibrinogen than those detected by commercial products (FIGS. 6 to 8).

Therefore, in one aspect,
the present invention is directed to a composition for blood coagulation testing containing a hemostatic enzyme and carboxymethyl chitosan (CMCS).

In the present invention, the hemostatic enzyme may be used without limitation as long as it is an enzyme that exhibits a hemostatic effect. Preferably, the hemostatic enzyme may be a native or recombinant thrombin-like enzyme (TLE) or a native or recombinant thrombin-like hemocoagulase (rTLH). More preferably, the hemostatic enzyme may be selected from the group consisting of thrombin, recombinant batroxobin, native batroxobin (nBat), Botropase, Ancrod, and Suring, but is not limited thereto.

As used herein, the term "recombinant batroxobin" refers to a batroxobin produced by modifying a native batroxobin cDNA sequence and expressing the modified cDNA in *Pichia pastoris.* A method for producing the same is described in detail in WO2009/084841, which is incorporated herein by reference.

As used herein, the term "BU" refers to a batroxobin unit, and "2 BU" refers to a batroxobin amount that coagulates plasma in 19 ± 0.2 seconds. The BU may be calculated by a titration method using a titration standard graph of native batroxobin (NIBSC).

In the present invention, the blood coagulation test may be used without limitation as long as it is a test related to a blood coagulation reaction. Preferably, the blood coagulation test may be selected from the group consisting of thrombin time (TT) measurement, Reptilase time measurement, and fibrinogen concentration measurement.

In the present invention, thrombin time measurement is a method of measuring the time taken for a clot to be formed after adding a standardized amount of thrombin to plasma. In this case, thrombin may be replaced with the composition of the present invention. When the composition of the present invention is used, it is not affected by anticoagulants such as heparin and aspirin.

In the present invention, Reptilase time measurement is a method of measuring the time taken for a clot to be formed after adding batroxobin to plasma. In this case, batroxobin may be replaced with the composition of the present invention. When the composition of the present invention is used, it is not affected by anticoagulants.

In the present invention, fibrinogen concentration may be determined using the Clauss method.

The Claus method is a method in which an excess of thrombin (35 to 200 U/ml) is added to a diluted sample plasma, the time of clot formation is measured, and the fibrinogen concentration in the sample is determined by comparison with a standard curve.

The standard curve is a graph showing the correlation between concentration and time determined by measuring the time of clot formation in a sample prepared by diluting a standard plasma containing a known concentration, and is given in units of g/l.

The blood coagulation test of the present invention may be performed by direct comparison and analysis using a generated standard curve, but is preferably performed using an automated device.

In the present invention, the automated device may be used without limitation as long as it may perform the blood coagulation test. Preferably, the automated device may be selected from the group consisting of ACL Family, ACL Futura, ACL Advance, ACL TOP Family, ACL10000 (Instrument Lab.), Thrombotimer (Behnk Electronik), Sysmex CA1500, CA660, CA620, CS5100, CS2500, CS1600 (Simens), STA-R evolution, STA-R Max, STA Compact Max, and STA Satellite (Stago), but is not limited thereto.

In the present invention, the recombinant hemostatic enzyme may be contained at a concentration of 0.1 to 100 BU/ml, preferably 0.5 to 90, 0.5 to 80, 1 to 70, 2 to 60, or 2.5 to 50 BU/ml, most preferably 50 BU/ml.

In the present invention, the carboxymethyl chitosan may be contained at a concentration of 0.1 to 100 mg/ml, preferably 0.5 to 30, 0.5 to 20, 0.5 to 15, 0.5 to 12, 0.5 to 10, 0.8 to 30, 0.8 to 20, or 0.8 to 15 mg/ml, most preferably 1 mg/ml.

In the present invention, the composition may further contain calcium chloride (CaCl₂) at a concentration of 0 to 100 mM, preferably 0.5 to 30, 0.5 to 20, 0.5 to 15, 0.5 to 12, 0.5 to 10, 0.8 to 30, 0.8 to 20, 0.8 to 15, or 0.8 to 12 mM, most preferably 10 mM.

In the present invention, the buffer may further contain 1 to 100 mM of buffer. The buffer may be at least one selected from the group consisting of 2-(N-morpholino)ethanesulfonic acid (MES), maleate, citrate, bis-tris, phosphate, N-(2-acetamido)iminodiacetic acid (ADA), carbonate, piperazine-N,N'-bis(2-ethanesulfonic acid (PIPES), N-(2-acetamido)-2-aminoethanesulfonic acid (ACES), 3-morpholino-2-hydroxypropanesulfonic acid (MOPSO), imidazole, bis-tris-propane, N,N-bis(2-hydroxyethyl)-2-aminoethanesulfonic acid (BES), 3-(N-morpholino)propanesulfonic acid (MOPS), N-[tris(hydroxymethyl)methyl]-2-aminoethanesulfonic acid (TES), 4-(2-hydroxyethyl)-1-piperazineethanesulfonic acid (HEPES), 3-bis(2-hydroxyethyl)amino-2-hydroxypropane-1-sulfonic acid (DIPSO), 3-[tris-(hydroxymethyl)methylamino]-2-hydroxypropane sulphonic acid (TAPSO), triethanolamine (TEA), N-(2-hydroxyethyl)-piperazine-N'-2-hydroxypropanesulfonic acid (HEPPSO), piperazine-1,4-bis(2-hydroxy-3-propanesulfonic acid (POPSO), tricine, glycylglycine, Tris, (3-[4-(2-hydroxyethyl)piperazin-1-yl]propane-1-sulfonic acid (HEPPS, EPPS), bicine, N-[tris(hydroxymethyl)methyl]-3-aminopropanesulfonic acid (TAPS), 2-amino-2-methyl-1,3-propanediol (AMPD), N-(1,1-dimethyl-2-hydroxyethyl)-3-amino-2-hydroxypropanesulfonic acid (AMPSO), taurine, and boric acid. Most preferably, the buffer may comprise 3-(N-morpholino)propanesulfonic acid (MOPS), but is not limited thereto.

In the present invention, the composition may further contain 0.1 to 10 mg/ml of a stabilizer.

In the present invention, the stabilizer may be at least one from among sugar alcohol, maltose, sorbitol, mannose, glucose, trehalose, albumin, lysine, glycine, gelatin, PEG, and Tween 80, but is not limited thereto.

The present invention is also directed to a method for measuring fibrinogen concentration, the method comprising steps of:
a) adding the composition to a serum-containing sample and measuring the rate of clot formation; and
b) determining the fibrinogen concentration from the rate of clot formation.

In the present invention, the serum-containing sample may be blood, preferably serum separated from blood, but is not limited thereto.

In another aspect, the present invention is directed to a method for screening an anticoagulant substance, the method comprising steps of:
a) adding a hemostatic enzyme and carboxymethyl chitosan to a serum-containing sample containing fibrinogen to obtain a mixture;
b) adding a candidate substance to the mixture;
c) measuring the time of clot formation; and
d) selecting the candidate substance as the anticoagulant substance when the time of clot formation increases compared to that in a control to which the candidate substance has not been added.

In the present invention, the candidate substance may be selected from among natural compounds, synthetic compounds, DNA, RNA, peptides, enzymes, ligands, cell extracts, or mammalian secretions. In addition, examples of the candidate substance include proteins, non-peptidic compounds, fermentation products, plant extracts, or plasma, and the compounds may be novel compounds or widely known compounds, and are preferably obtained from a library of synthetic or natural compounds.

Methods for obtaining these libraries of compounds are known in the art. Libraries of synthetic compounds are commercially available from Maybridge Chemical Co. (UK), Comgenex (USA), Brandon Associates (USA), Microsource (USA) and Sigma-Aldrich (USA), and libraries of natural compounds are commercially available from Pan Laboratories (USA) and MycoSearch (USA). The sample may be obtained using any of numerous combinatorial library methods known in the art, for example, biological libraries, spatially addressable parallel solid phase or solution phase libraries, synthetic library methods requiring deconvolution, the "one-bead one-compound" library method, and synthetic library methods using affinity chromatography selection. Methods for the synthesis of molecular libraries are disclosed in DeWitt et al., Proc. Natl. Acad. Sci. U.S.A. 90, 6909, 1993; Erb et al. Proc. Natl. Acad. Sci. U.S.A. 91, 11422, 1994; Zuckermann et al., J. Med. Chem. 37, 2678, 1994; Cho et al., Science 261, 1303, 1993; Carell et al., Angew. Chem. Int. Ed. Engl. 33, 2059, 1994; Carell et al., Angew. Chem. Int. Ed. Engl. 33, 2061; Gallop et al., J. Med. Chem. 37, 1233, 1994, and the like.

### Examples

Hereinafter, the present invention will be described in detail with reference to examples. It will be obvious to those skilled in the art that these examples serve merely to illustrate the present invention, and the scope of the present invention is not construed as being limited by these examples.

### Example 1: Construction, Isolation and Purification of Recombinant Batroxobin Expression Vector

According to the method disclosed in Korean Patent No. 10-1801150, a recombinant batroxobin expression vector was constructed, isolated and purified. Then, the expression vector stored in a refrigerator (2 to 8°C) and used in subsequent analysis.

### Example 2: Evaluation of Synergistic Effect of Recombinant Batroxobin and Carboxymethyl Chitosan

### 2-1. Measurement of Plasma Clotting Time

Human plasma (Innovative Research, USA) was purchased, dispensed 1 ml each and then frozen. At the time of measurement, the plasma was thawed quickly at 37°C and used.

As samples for analysis, rTLH and native batroxobin (Hyphen, USA), thrombin (Sigma, USA), Botropase (Ravizza, Italy), Ancrod (Knoll Pharmaceutical, USA), Suling (Konruns, China) were each diluted with saline to a concentration of 10 BU/ml (based on native batroxobin), and then diluted with 10 mM Tris-HCl (pH 6.8), CMCS or CS (chitosan) to a concentration of 5 BU/ml.

Each sample and plasma were preheated at 37°C for 10 minutes, and then 100 µl of each sample was added to 300 µl of the plasma, and the clotting time of the plasma was measured with a Thrombotimer.

As a result, as shown in FIG. 1, it was confirmed that the plasma clotting time of rTLH containing carboxymethyl chitosan was shorter than that of chitosan-containing rTLH or the composition containing only rTLH.

### 2-2. Measurement of Fibrinogen Clotting Time

Fibrinogen (Sigma, USA) was purchased and dissolved at a concentration of 2 mg/ml.

Samples for analysis were prepared in the same manner as the compositions for measuring the plasma clotting time.

The sample and fibrinogen were placed in a machine according to the FIB-C protocol of ACL10000, and the fibrinogen clotting time was measured according to the program.

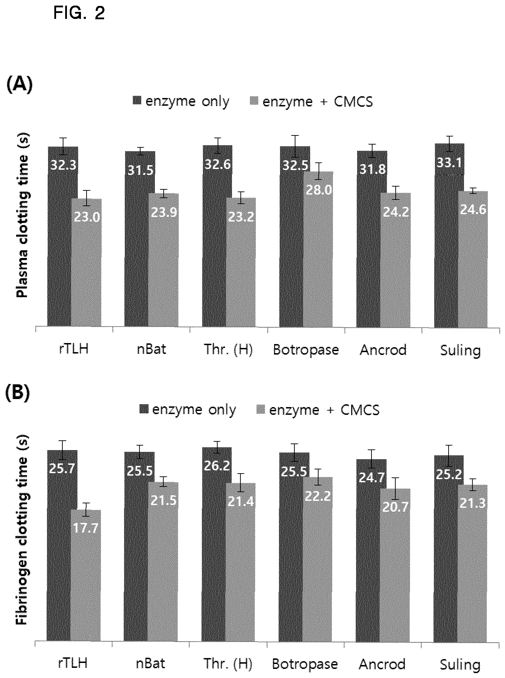

As a result, as shown in FIGS. 1 and 2, it was confirmed that the fibrinogen clotting time of the rTLH + CMCS composition was shorter than that of rTLH only or rTLH + CS (chitosan).

### 2-3. Measurement of Fibrinogen α-Chain Cleavage Time

Human fibrinogen was dissolved at a concentration of 2 mg/mL, buffer or CMCS was added thereto and mixed well therewith, and then rTLH was added thereto and allowed to react. The reaction time was 10 to 30 seconds. After completion of the reaction, 5X SDS-PAGE sample buffer was immediately added and the reaction was terminated by boiling at 100°C for 5 minutes. Samples were loaded on a 12% SDS-PAGE gel and electrophoresed for 2 hours, and the gel was stained with Coomassie blue.

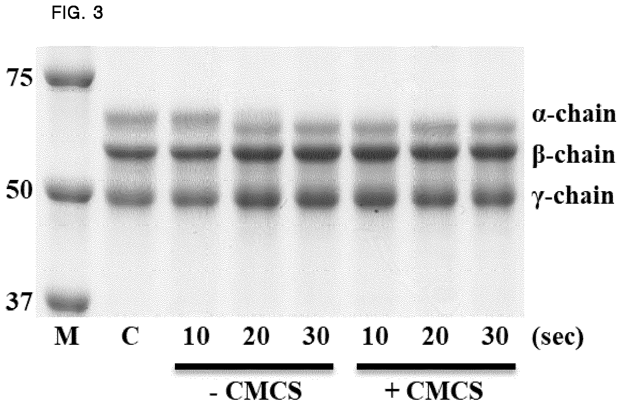

As a result, as shown in FIG. 3, it was confirmed that, when CMCS was not added, the fibrinogen α-chain was completely cleaved in 30 seconds, and when CMCS was added, the fibrinogen α-chain was completely cleaved in 10 seconds.

### 2-4. Measurement of Enzymatic Activity

The thrombin substrate S-2238 (Hyphen, USA) was purchased and dissolved at a concentration of 20 mM. The rTLH activity was measured at final concentrations of 25, 50, 100, 200 and 400 µM, and 100 mM Tris-HCl (pH 6.8) was added for dilution.

For preparation of samples for analysis, rTLH was diluted to a concentration of 10 BU/ml and mixed with 10 mM Tris-HCl (pH 6.8) or CMCS.

20 µl of each sample composed of 5 BU/ml rTLH or 5 BU/ml rTLH + 10 mg/ml CMCS was added to each well of a 96-well plate, and 180 µl of the thrombin substrate was added to each well at various concentrations. Then, the absorbance of each well was measured using ELISA at 37°C for 20 minutes.

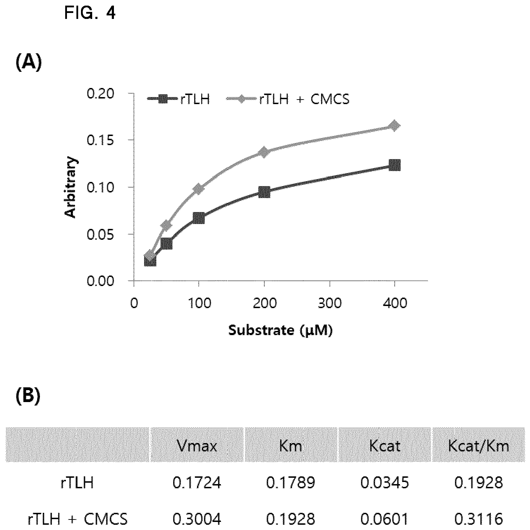

As a result, as shown in FIG. 4, it was confirmed that CMCS increased the enzymatic activity of rTLH.

### 2-5. Measurement of Clot Strength

Rat whole blood was purchased and used.

For preparation of samples for analysis, rTLH was diluted to a concentration of 5 BU/ml.

20 µl of CMCS was added to 300 µl of rat whole blood at various concentrations (0.1, 0.25 and 0.5 mg/ml), and then 20 µl of rTLH and 20 µl of CaCl₂ were added to 300 µl of the blood. The clot strength was measured using ROTEM for 1 hour.

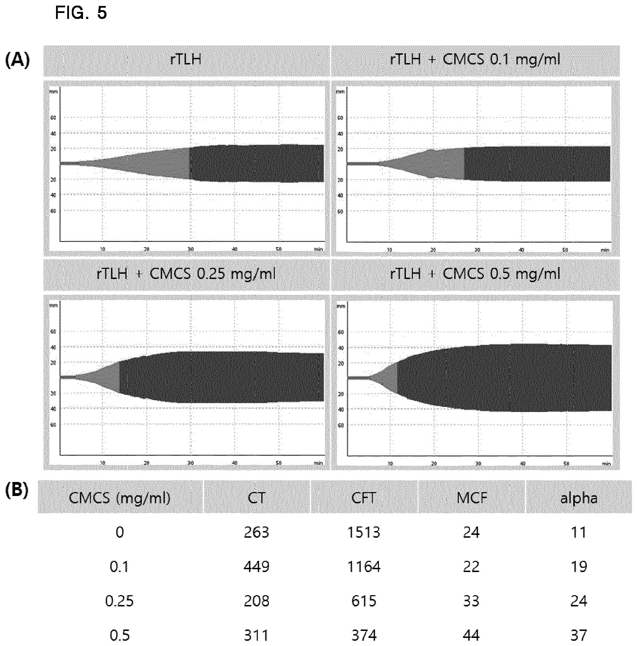

As a result, as shown in FIG.5, it was confirmed that the clot strength increased depending on the concentration of CMCS.

### Example 3: Fibrinogen Concentration Measurement Based on Mixture of Recombinant Batroxobin and Carboxymethyl Chitosan

Detection of various concentrations of fibrinogen using a reagent containing 2.5 BU/ml or 5 BU/ml of the recombinant batroxobin produced in Example 1 and 10 mg/ml of carboxymethyl chitosan was attempted using the ACL 10000 FIB_C protocol.

As a result, as shown in FIG. 6, it was confirmed that 0.3 mg/ml of fibrinogen was detectable.

In addition, detection of various concentrations of fibrinogen was performed using 10 mM calcium chloride (CaCl₂) in addition to the above-described conditions. As a result, as shown in FIG. 7, it could be confirmed that 0.2 mg/ml of fibrinogen was detectable.

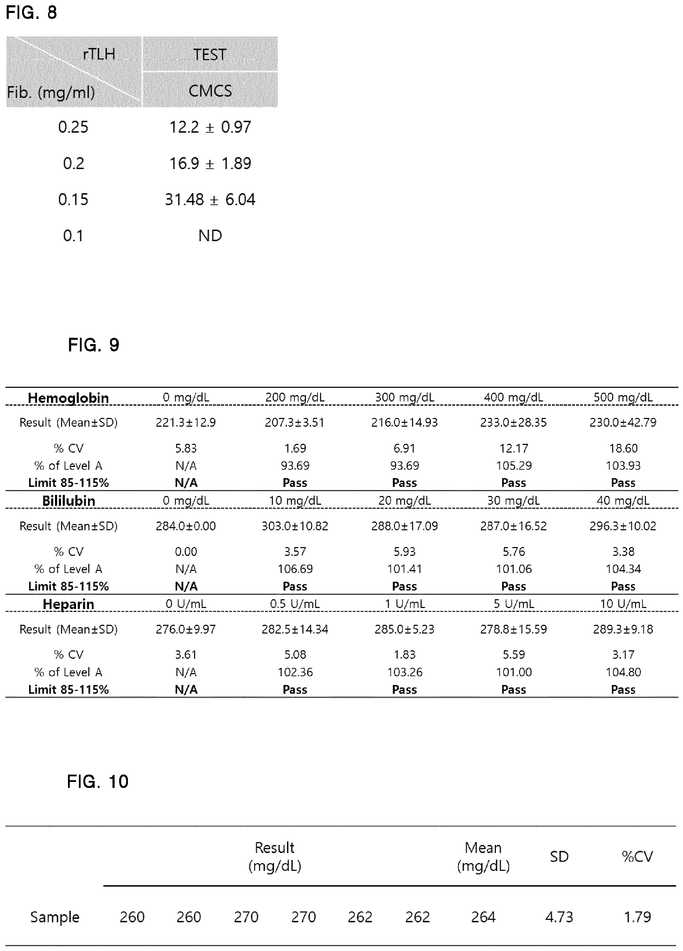

In addition, it was confirmed that the serum concentration of fibrinogen detectable using the composition was up to 0.15 mg/ml (FIG. 8), which was significantly lower than the detection limit of the best commercial fibrinogen detection reagent (0.35 mg/ml).

### Example 4: Interference Performance Test for Fibrinogen Concentration Measurement Reagent Based on Mixture of Recombinant Batroxobin and Carboxymethyl Chitosan

In order to check whether there is interference of the measured values in abnormal blood and blood containing anticoagulants, interference performance was tested. Calibration plasma was placed in a machine according to the FIB-C protocol of ACL TOP 300 and then a calibration curve was made by performing measurement according to the program. Hemoglobin, bilirubin and heparin were used as interfering substances, and each reagent was dissolved at high concentration and added to plasma at various concentrations, followed by measurement.

As a result, as shown in FIG. 9, hemoglobin had no effect on the measurement result value up to a concentration of 500 mg/dL, and bilirubin had no effect on the measurement result value up to a concentration of 40 mg/dL. In particular, it could be confirmed that the anticoagulant heparin had no effect on the measured value up to a concentration of 10 U/mL, indicating that the composition of the present invention had lower interference than the best commercial fibrinogen detection reagent having an interference limit of 1 U/mL.

### Example 5: Accuracy Evaluation Test for Fibrinogen Concentration Measurement Reagent Based on Mixture of Recombinant Batroxobin and Carboxymethyl Chitosan

A test was performed to confirm the degree of coincidence between the fibrinogen concentration value measured using the prepared reagent and the true value. The test was performed using an international standard material having a recognized reference value. As the standard material, the NIBSC 3^{rd} international standard for fibrinogen plasma (09/264) was used. The standard material and the reagent were placed according to the protocol of ACL TOP system, and then measurement was performed according to the program.

As a result, as shown in FIG. 10, it could be confirmed that the result value measured using the composition showed a difference of ±3% or less from the result value of the international standard material, indicating that the composition has significantly better accuracy than the best commercial fibrinogen detection reagent having a difference of ±15% or less from the result value of the international standard material.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The composition for blood coagulation testing according to the present invention is useful because it may perform a blood coagulation test with high sensitivity and high speed by improving the activity of the hemostatic enzyme and clot strength and increasing the rate of fibrin formation.

## Claims

1. A composition for blood coagulation testing containing a hemostatic enzyme and carboxymethyl chitosan (CMCS).

2. The composition of claim 1, wherein the blood coagulation testing is selected from the group consisting of thrombin time (TT) measurement, Reptilase time measurement and fibrinogen concentration measurement.

3. The composition of claim 1, wherein the hemostatic enzyme is selected from the group consisting of thrombin, recombinant batroxobin, native batroxobin, Botropase, Ancrod, and Suling.

4. The composition of claim 1, wherein the hemostatic enzyme is contained at a concentration of 0.1 to 100 BU/ml.

5. The composition of claim 1, wherein the carboxymethyl chitosan is contained at a concentration of 0.1 to 100 mg/ml.

6. The composition of claim 1, further containing 0 to 100 mM of calcium chloride (CaCl₂).

7. The composition of claim 1, further containing 1 to 100 mM of a buffer.

8. The composition of claim 7, wherein the buffer is at least one selected from the group consisting of 2-(N-morpholino)ethanesulfonic acid (MES), maleate, citrate, bis-tris, phosphate, N-(2-acetamido)iminodiacetic acid (ADA), carbonate, piperazine-N,N'-bis(2-ethanesulfonic acid (PIPES), N-(2-acetamido)-2-aminoethanesulfonic acid (ACES), 3-morpholino-2-hydroxypropanesulfonic acid (MOPSO), imidazole, bis-tris-propane, N,N-bis(2-hydroxyethyl)-2-aminoethanesulfonic acid (BES), 3-(N-morpholino)propanesulfonic acid (MOPS), N-[tris(hydroxymethyl)methyl]-2-aminoethanesulfonic acid (TES), 4-(2-hydroxyethyl)-1-piperazineethanesulfonic acid (HEPES), 3-bis(2-hydroxyethyl)amino-2-hydroxypropane-1-sulfonic acid (DIPSO), 3-[tris-(hydroxymethyl)methylamino]-2-hydroxypropane sulphonic acid (TAPSO), triethanolamine (TEA), N-(2-hydroxyethyl)-piperazine-N'-2-hydroxypropanesulfonic acid (HEPPSO), piperazine-1,4-bis(2-hydroxy-3-propanesulfonic acid (POPSO), tricine, glycylglycine, Tris, (3-[4-(2-hydroxyethyl)piperazin-1-yl]propane-1-sulfonic acid (HEPPS, EPPS), bicine, N-[tris(hydroxymethyl)methyl]-3-aminopropanesulfonic acid (TAPS), 2-amino-2-methyl-1,3-propanediol (AMPD), N-(1,1-dimethyl-2-hydroxyethyl)-3-amino-2-hydroxypropanesulfonic acid (AMPSO), taurine, and boric acid.

9. The composition of claim 1, further containing 0.1 to 10 mg/ml of a stabilizer.

10. The composition of claim 9, wherein the stabilizer is at least one selected from among sugar alcohol, maltose, sorbitol, mannose, glucose, trehalose, albumin, lysine, glycine, gelatin, PEG, and Tween 80.

11. A method for measuring fibrinogen concentration, the method comprising steps of:
a) adding the composition of any one of claims 1 to 10 to a serum-containing sample and measuring a rate of clot formation; and
b) determining the fibrinogen concentration from the rate of clot formation.

12. A method for screening an anticoagulant substance, the method comprising steps of:
a) adding a hemostatic enzyme and carboxymethyl chitosan to a serum-containing sample containing fibrinogen to obtain a mixture;
b) adding a candidate substance to the mixture;
c) measuring a time of clot formation; and
d) selecting the candidate substance as the anticoagulant substance when the time of clot formation increases compared to that in a control to which the candidate substance has not been added.
